(19) Europäisches Patentamt
European Patent Office
Office européen des brevets

(11) Veröffentlichungsnummer: **0 064 180**
**B1**

(12) # EUROPÄISCHE PATENTSCHRIFT

(45) Veröffentlichungstag der Patentschrift: **27.06.84**

(51) Int. Cl.³: **C 07 C 31/20**, C 07 C 29/04

(21) Anmeldenummer: **82103109.3**

(22) Anmeldetag: **13.04.82**

(54) **Verfahren zur Herstellung von 2,3-Dimethylbutan-2,3-diol.**

(30) Priorität: **24.04.81 DE 3116294**

(43) Veröffentlichungstag der Anmeldung: **10.11.82 Patentblatt 82/45**

(45) Bekanntmachung des Hinweises auf die Patenterteilung: **27.06.84 Patentblatt 84/26**

(84) Benannte Vertragsstaaten: **BE CH DE FR GB IT LI NL**

(56) Entgegenhaltungen:
**EP - A - 0 010 236**

**Patent Abstracts of Japan Band 2, Nr. 105, 30. August 1978 Seite 1857C78**

(73) Patentinhaber: **BAYER AG, Konzernverwaltung RP Patentabteilung, D-5090 Leverkusen 1 Bayerwerk (DE)**

(72) Erfinder: **Wilms, Klaus Günter, Dr., Siegstrasse 3, D-4047 Dormagen-1 (DE)**
Erfinder: **Waldmann, Helmut, Dr., H.T.-von Böttinger-Strasse 15, D-5090 Leverkusen 1 (DE)**
Erfinder: **Grolig, Johann, Dr., Heinrich-Lübke-Strasse 22, D-5090 Leverkusen 1 (DE)**

**EP 0 064 180 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von 2,3-Dimethylbutan-2,3-diol aus 2,3-Dimethylbutenen und Sauerstoff oder sauerstoffhaltigen Gasen.

2,3-Dimethylbutan-2,3-diol ist ein wichtiges Zwischenprodukt, das beispielsweise für Kautschuksynthesen verwendet werden kann (siehe Römpps Chemie Lexikon, Stuttgart 1974, Seite 2701).

Es sind schon eine Reihe von Verfahren zur Herstellung von 2,3-Dimethylbutan-2,3-diol bekanntgeworden, die jedoch alle nicht befriedigend sind.

So kann man 2,3-Dimethylbutan-2,3-diol durch Reduktion von Aceton herstellen. Diese Reduktion kann mit Magnesium in Gegenwart von Quecksilberchlorid (siehe R. E. W. Adams, Org. Synth. 5, 87; Coll. Vol. I (1956) 459), mit Aluminiumamalgam oder elektrochemisch (siehe DE-PS 306 304 und 310 023) durchgeführt werden. Soweit hierbei in Gegenwart von Quecksilber oder Quecksilberverbindungen gearbeitet wird, ist dessen Giftigkeit und das Entstehen von quecksilberhaltigen Nebenprodukten nachteilig (siehe Beilstein, E II, 1, 553). Bei der Reduktion mit Aluminiumamalgam entsteht außerdem Isopropylalkohol als Nebenprodukt (siehe L. F. und M. Fieser, Organische Chemie, Verlag Chemie, Weinheim (1965), Seite 509) und die elektrochemische Reduktion ist sehr energieintensiv.

Ein anderes Verfahren geht ebenfalls von Aceton aus, das photochemisch in Gegenwart von Wasserstoffdonatoren (z. B. Isopropanol) in 2,3-Dimethylbutan-2,3-diol überführt wird (siehe G. O. Schenk, Dechema-Monographie 24, 105 (1955)). Nach 24 Stunden Reaktionszeit beträgt die Ausbeute aber nur 4% der Theorie (siehe Houben-Weyl, Methoden der organischen Chemie, Band IV/Ia, Teil 2, Georg Thieme Verlag Stuttgart (1980), Seite 1499).

Weitere Verfahren gehen aus von 2,3-Dimethylbutenen. So wird beschrieben, daß man aus 2,3-Dimethyl-2-buten durch Behandlung mit wäßriger Kaliumpermanganat-Lösung 2,3-Dimethylbutan-2,3-diol erhalten kann (siehe Couturier, Annales de Chimie et de Physique, 6. Serie, 26, (1882), Seite 479). Wegen der Verwendung von Kaliumpermanganat ist dies jedoch keine günstige Methode, um im technischen Maßstab 2,3-Dimethylbutan-2,3-diol herzustellen.

Es ist auch bekannt, daß man 2,3-Dimethylbutene durch Umsetzung mit Wasserstoffperoxid und Ameisensäure in 2,3-Dimethylbutan-2,3-diol überführen kann (siehe DE-AS 2 844 637). Bezogen auf eingesetztes Wasserstoffperoxid beträgt die Selektivität der Diolbildung nur ca 66%, d. h. 34% des eingesetzten teueren Wasserstoffperoxids tragen nicht zur Diolbildung bei. Durch den Einsatz von Ameisensäure, die ein sehr aggressives Agens ist, treten Korrosionsprobleme auf. Außerdem bildet sich bei diesem Verfahren zunächst das Monoformiat des 2,3-Dimethylbutan-2,3-diols aus dem das Diol erst durch Verseifung mit wäßrigem Alkalihydroxid freigesetzt werden kann. Es handelt sich also um ein mehrstufiges Verfahren mit dem zusätzlichen Nachteil, daß bei der Verseifung Abwässer anfallen, die Alkaliformiate enthalten.

Weiterhin wird beschrieben, daß bei der Umsetzung von 2,3-Dimethyl-2-buten ( = Tetramethylethylen) mit Sauerstoff in Gegenwart von

$$trans-MCl(CO)(PH_3P)_2$$

(M = Rhodium oder Iridium, Ph = Phenyl) als Katalysator ein Gemisch aus 2,3-Dimethyl-2,3-epoxi-butan, 2,3-Dimethyl-3-hydroxybuten-1 und 2,3-Dimethyl-3-hydroperoxybuten-1 erhalten werden kann (siehe J. Org. Chem. 37, 2881 bis 2884 (1972)). Eine Bildung von 2,3-Dimethylbutan-2,3-diol tritt nicht ein. Ohne Katalysator erfolgt praktisch kein Umsatz von 2,3-Dimethyl-2-buten (siehe a. a. O. Seite 2882, Tabelle I, 1. Zeile).

Schließlich ist aus der JP-OS 65 087/78 (= japanische Patentanmeldung 140 890/76) bekannt, daß man Diole herstellen kann, indem man in flüssiger Phase eine monoolefinische Kohlenwasserstoff-Fraktion in Gegenwart der 0,7- bis 8fachen Gewichtsmenge Wasser (bezogen auf die Kohlenwasserstoff-Fraktion) bei Temperaturen von 80 bis 200°C und Drucken von 5 bis 100 kg/cm$^2$ mit Sauerstoff oder einem sauerstoffhaltigen Gas oxidiert. Die Reaktionszeiten betragen bei diesem Verfahren über 6 und bis zu 13 Stunden und gute Ergebnisse werden auch dann nur erhalten, wenn man Katalysatoren zusetzt. 2,3-Dimethylbutene sind diesem Verfahren nicht unterworfen worden, und im Hinblick auf die vorgenannte Literaturstelle J. Org. Chem. 37, 2881 bis 2884 war nicht zu erwarten, daß 2,3-Dimethylbutene mit Sauerstoff in 2,3-Dimethylbutan-2,3-diol umgewandelt werden können.

Es besteht also immer noch das Bedürfnis nach einem einfachen und effektiven Verfahren zur Herstellung von 2,3-Dimethylbutan-2,3-diol.

Es wurde nun ein Verfahren zur Herstellung von 2,3-Dimethylbutan-2,3-diol gefunden, das dadurch gekennzeichnet ist, daß man 2,3-Dimethylbutene mit Sauerstoff oder einer Sauerstoff enthaltenden Gas in Gegenwart von 0,01 bis 20 Gew.-Teilen Wasser (bezogen auf eingesetzte 2,3-Dimethylbutene) bei Temperaturen von 40 bis 180°C und Verweilzeiten von 5 bis 150 Minuten ohne Zusatz und Katalysatoren unsetzt.

Als in das erfindungsgemäße Verfahren einsetzbare 2,3-Dimethylbutene kommen beispielsweise 2,3-Dimethyl-2-buten, 2,3-Dimethyl-1-buten, beliebige Gemische aus 2,3-Dimethyl-2-buten und 2,3-Dimethyl-1-buten und beliebige Gemische aus 2,3-Dimethyl-2-buten und/oder 2,3-Dimethyl-1-buten mit Inerten in Frage. Solche Inerte können beispielsweise Kohlenwasserstoffe und chlorierte Kohlenwasserstoffe der aliphatischen, cycloaliphatischen und aromatischen Reihe sein, beispielsweise gesättigte aliphatische Kohlenwasserstoffe wie n-Hexan oder Dimethylbutan, gesättigte cycloaliphatische Kohlenwasserstoffe, wie Decalin, aromatische Kohlenwasserstoffe, wie Benzol, Toluol oder Xylol oder chlorierte Kohlenwasserstoffe, beispielsweise chlorierte Aromaten. Vorzugsweise sind solche Inerte n-Hexan, Dimethylbutan, Benzol oder chlorierte Kohlenwasserstoffe.

2,3-Dimethyl-2-buten und 2,3-Dimethyl-1-buten sind leicht zugängliche Ausgangsstoffe, die man beispielsweise durch Dimerisierung von Propylen erhalten (siehe Houben-Weyl, Methoden der organischen Chemie, Band V/Ib, Seite 550 bis 551, Stuttgart (1972)) und dann sofort oder gegebenenfalls nach vorheriger Destillation in das erfindungsgemäße Verfahren einsetzen kann. Auch durch Dehydratisieren von 2,3-Dimethylbutan-2-ol können 2,3-Dimethylbutene erhalten werden, die in das erfindungsgemäße Verfahren eingesetzt werden können.

Vorzugsweise werden in das erfindungsgemäße Verfahren 2,3-Dimethyl-2-buten oder 2,3-Dimethyl-2-buten enthaltende Gemische der vorgenannten Art eingesetzt, die über 60%, vorzugsweise über 70% 2,3-Dimethyl-2-buten enthalten.

Der Sauerstoff kann dem erfindungsgemäßen Verfahren als reiner Sauerstoff oder in Form eines Sauerstoff enthaltenden Gasgemisches zugeführt werden. Wenn ein Sauerstoff enthaltendes Gasgemisch eingesetzt wird, enthält dieses neben Sauerstoff vorzugsweise nur noch inerte Bestandteile. Solche inerten Bestandteile können beispielsweise Stickstoff, Kohlendioxid und/oder Edelgase, wie Helium, Neon, Argon und/oder Krypton sein. Man kann auch ein, beispielsweise durch Verbrennung von Methan mit Luft herstellbares, Inertgasgemisch aus Kohlendioxid und Stickstoff als inerten Bestandteil des Sauerstoff enthaltenden Gasgemisches verwenden. Auch Zusätze von Ozon oder atomarem Sauerstoff sind möglich.

Vorzugsweise enthalten die Sauerstoff enthaltenden Gasgemische zwischen 10 und über 99% Sauerstoff. Zum Einsatz in das erfindungsgemäße Verfahren verwendet man vorzugsweise Luft, Sauerstoff enthaltende Gasgemische, die über 50% Sauerstoff enthalten oder reinen Sauerstoff.

Es ist vorteilhaft, den einzusetzenden Sauerstoff in mehreren Teilmengen nacheinander zuzugeben, beispielsweise indem man während der Umsetzung mehrmals Sauerstoff und/oder Sauerstoff enthaltende Gase aufdrückt.

Wenn ein Sauerstoff enthaltendes Gasgemisch eingesetzt wird, können die verschiedenen Komponenten des Gasgemisches gemeinsam, getrennt oder in geeigneten Kombinationen zusammengefaßt der Reaktionszone zugeführt werden. Die Vermischung der Gaskomponenten kann innerhalb oder außerhalb der Reaktionszone erfolgen. Die Vermischung der Gaskomponenten außerhalb der Reaktionszone kann beispielsweise im Freistrahl, in Kanälen, in Mischapparaturen oder in Mischkammern erfolgen. Geeignet sind beispielsweise Injektormischer und Strahlmischer. Für die Gasvermischung können auch Rohrorgane eingesetzt werden. Auch Gebläse können gleichzeitig als Mischvorrichtung dienen.

Das erfindungsgemäße Verfahren wird in Gegenwart von 0,01 bis 20 Gew.-Teilen Wasser (bezogen auf 1 Gew.-Teil eingesetzte 2,3-Dimethylbutene) durchgeführt. Vorzugsweise werden 0,1 bis 15, besonders bevorzugt 0,2 bis 10 Gew.-Teile Wasser, bezogen auf 1 Gew.-Teil eingesetzte 2,3-Dimethylbutene, eingesetzt.

Das Wasser kann flüssig oder dampfförmig der Reaktionszone zugeführt werden. Vorzugsweise setzt man das Wasser flüssig zu. Weiterhin ist es bevorzugt, entsalztes Wasser zu verwenden, um Korrosionsprobleme zu vermeiden. In geeigneten Apparaturen kann man aber auch mit nicht oder nur teilweise entsalztem Wasser arbeiten, beispielsweise mit sogenanntem Betriebswasser. In manchen Fällen ist es vorteilhaft mit basisch reagierenden Verbindungen versetztes Wasser zu verwenden, beispielsweise dann, wenn man während der Reaktion die Bildung sauer reagierender Nebenprodukte beobachtet. Geeignete basisch reagierende Verbindungen sind beispielsweise Alkali- und/oder Erdalkalicarbonate, -hydrogencarbonate, -oxide und -hydroxide, wie Natriumhydrogencarbonat, Calciumcarbonat, Kaliumhydroxid und Magnesiumoxid, aber auch organische basisch reagierende Verbindungen, beispielsweise Amine.

Das erfindungsgemäße Verfahren wird bei Temperaturen von 40 bis 180°C durchgeführt. Vorzugsweise arbeitet man bei 60 bis 160°C, besonders bevorzugt bei 80 bis 140°C.

Neben der Arbeitsweise unter isothermen Bedingungen, d. h. Einhaltung einer einheitlichen Temperatur während der gesamten Reaktion, kann man die Umsetzung auch unter Ausbildung eines sogenannten Temperaturgradienten durchführen, d. h. bei im Verlauf der Reaktion ansteigender oder abfallender Temperatur.

Das erfindungsgemäße Verfahren wird so durchgeführt, daß die Verweilzeit (bei diskontinuierlicher Arbeitsweise) bzw. die mittlere Verweilzeit (bei kontinuierlicher Arbeitsweise) 5 bis 150 Minuten beträgt. Vorzugsweise beträgt diese Zeit 8 bis 120 Minuten, besonders bevorzugt 10 bis 70 Minuten. Innerhalb dieser Grenzen ist die Verweilzeit relativ groß, wenn Sauerstoff oder Sauerstoff enthaltende Gase diskontinuierlich zugeführt werden.

Das erfindungsgemäße Verfahren wird ohne den Zusatz von Katalysatoren durchgeführt.

Der Druck kann beim erfindungsgemäßen Verfahren in weiten Grenzen variieren. Es kann beispielsweise bei einem Systemdruck von 0,9 bis 90 bar durchgeführt werden. Vorzugsweise beträgt dieser Druck 2,0 bis 70 bar, besonders bevorzugt 5,0 bis 50 bar. In Sonderfällen können die angegebenen Druckbereiche auch unter- oder überschritten werden. Der Druck wird vorzugsweise eingestellt, indem man den Sauerstoff oder das Sauerstoff enthaltende Gas mit dem entsprechenden Druck aufdrückt. Vorteilhaft ist es, während der Reaktion mehrmals Sauerstoff oder sauerstoffhaltiges Gas ausfzudrükken.

Weiterhin ist es vorteilhaft, während des Ablaufs der Reaktion für eine gute Durchmischung der Reaktionskomponenten zu sorgen, beispielsweise durch intensives Rühren oder Verwendung eines Begasungsrührers.

Das erfindungsgemäße Verfahren kann innerhalb der angegebenen Parameter so durchgeführt werden, daß der Umsatz von 2,3-Dimethylbutenen innerhalb weiter Grenzen variieren kann, z. B. zwischen 1 und 95%. Vorzugsweise beträgt dieser Umsatz am Ende der Reaktion 20 bis 90%, besonders bevorzugt 30 bis 85%.

Bei 2,3-Dimethylbuten-Umsätzen von 30 bis 85% erzielt man nach dem erfindungsgemäßen Verfahren 2,3-Dimethylbutan-2,3-diol-Selektivitäten von beispielsweise 70 bis 80%. Das erfindungsgemäße Verfahren kann auch mit 2,3-Dimethylbuten-Umsätzen von größer als 85% durchgeführt werden, doch nimmt die 2,3-Dimethylbutan-2,3-diol-Selektivität bei höheren Umsätzen unter Umständen etwas ab.

Das erfindungsgemäßen Verfahren kann sowohl in der Flüssig- als auch in der Gasphase durchgeführt werden. Vorzugsweise wird es in der Flüssigphase durchgeführt.

Die Durchführung des erfindungsgemäßen Verfahrens kann diskontinuierlich oder kontinuierlich in dem für Umsetzungen dieser Art üblichen Vorrichtungen erfolgen. Beispielsweise sind Rührkessel, Siedereaktoren, Röhrenreaktoren, Schlaufenreaktoren, Schleifenreaktoren, Rührkesselkaskaden und Blasensäulen geeignet.

Als Werkstoffe für die Apparate zur Durchführung des erfindungsgemäßen Verfahrens können verschiedene Materialien verwendet werden. Beispielsweise sind Glas, Edelstahl, Nickellegierungen, Zirkon, Tantal und emaillierte Materialien geeignet.

Die Reaktionswärme kann auf verschiedene Weise abgeführt werden, beispielweise durch innen- oder außenliegende Kühler oder durch Sieden unter Rückfluß, z. B. in Siedereaktoren.

Die 2,3-Dimethylbutene oder sie enthaltende Gemische können auf verschiedene Art in die für die Umsetzung vorgesehene Vorrichtung eingebracht werden. Man kann sie gemeinsam mit oder getrennt von dem Wasser der Reaktionszone zuführen. Weiterhin ist es möglich die 2,3-Dimethylbutene oder sie enthaltenden Gemische und das Wasser an verschiedenen Stellen der Reaktionszone zuzuführen. Bei Verwendung mehrerer als Kaskade angeordneter Reaktoren kann es vorteilhaft sein, die 2,3-Dimethylbutene oder sie enthaltende Gemische ausschließlich dem ersten Reaktor zuzuführen. Man kann ihre Zugabe aber auch auf verschiedene oder alle Reaktoren aufteilen.

Bei Verwendung mehrerer als Kaskade angeordneter Reaktoren kann man Sauerstoff oder Sauerstoff enthaltende Gasgemische und/oder Wasser getrennt oder zusammen in jeden oder nur einige der Kessel der Kaskade geben.

Die Aufarbeitung der nach Durchführung des erfindungsgemäßen Verfahrens vorliegenden Reaktionsgemische und die Abtrennung des erhaltenen 2,3-Dimethylbutan-2,3-diols kann auf verschiedene Weise erfolgen. Wenn bei Temperaturen oberhalb des Siedepunktes (bei Normaldruck) der 2,3-Dimethylbutene und bei erhöhtem Druck gearbeitet wurde, ist es zweckmäßig, zunächst auf Temperaturen unterhalb des Siedepunktes (bei Normaldruck) der 2,3-Dimethylbutene abzukühlen, den Druck zu entspannen und dann das 2,3-Dimethylbutan-2,3-diol abzutrennen. Es ist unter Umständen vorteilhaft, die Druckentspannung unter Einleitung von inerten Gasen, wie Stickstoff, Kohlendioxid oder Edelgasen, vorzunehmen, um die Bildung explosionsfähiger Gasgemische auszuschließen. Das bei der Druckentspannung freiwerdende Gasgemisch kann, gegenbenenfalls nach Reinigung und Anreicherung mit Sauerstoff, wieder in die Reaktion zurückgeführt werden.

Beispielsweise kann man das Reaktionsgemisch nach Druckentspannung zunächst auf Temperaturen oberhalb des Schmelzpunkts von 2,3-Dimethylbutan-2,3-diol-hydrat abkühlen, d. h. auf Temperaturen oberhalb ca. 48°C, beispielsweise auf 50 bis 70°C, vorzugsweise auf 50 bis 60°C, besonders bevorzugt auf 50 bis 55°C, und so ein flüssiges 2-Phasen-Gemisch erhalten, eine obere organische Phase, die im wesentlichen unumgesetzte 2,3-Dimethylbutene enthält und eine untere wäßrige Phase, die im wesentlichen 2,3-Dimethylbutan-2,3-diol und Wasser enthält. Die so erhältliche organische Phase kann man in die Reaktion zurückführen, gegebenenfalls nachdem man sie ganz oder teilweise einer Destillationsstufe zur Anreicherung der 2,3-Dimethylbutene zugeführt hat. Diese Anreicherung kann auch durch Rektifikation, beispielsweise in einer Bodenkolonne, erfolgen. Die Destillation oder Rektifikation kann bei Normaldruck, erhöhtem Druck oder erniedrigtem Druck erfolgen. Die erhaltene wäßrige Phase kann man dann weiter abkühlen, beispielsweise auf Temperaturen unterhalb 48°C bis —10°C, vorzugsweise auf 30 bis —5°C, besonders bevorzugt auf 20 bis 0°C. Diese Abkühlung kann auf verschiedene Weise erfolgen, beispielsweise durch äußere Kühlung oder durch Vakuumkühlung, z. B. in Form einer Vakuumkristallisation. Das dabei, gegebenenfalls nach Animpfen, sich kristallin abscheidende 2,3-Dimethylbutan-2,3-diol-hydrat kann auf beliebige Weise abgetrennt werden, z. B. durch Filtration, Dekantieren, Sedimentieren, Zentrifugieren oder Abpressen. Die verbleibe Mutterlauge kann, gegebenenfalls nach Zusatz von Frischwasser, ganz oder teilweise in die Reaktion zurückgeführt werden, gegebenenfalls nach Abdestillieren von tiefer als Wasser siedenden organischen Bestandteilen und gegebenenfalls nach Neutralisation und/oder Reinigung mit einem Ionenaustauscher.

Beispielsweise kann das Reaktionsgemisch

nach Druckentspannung aber auch direkt auf Temperaturen unterhalb des Schmelzpunktes von 2,3-Dimethylbutan-2,3-diol-hydrat, d. h. auf Temperaturen unterhalb ca. 48° C, abgekühlt und so kristallines 2,3-Dimethylbutan-2,3-diol-hydrat und mindestens eine, gegebenenfalls auch zwei flüssige Phasen erhalten werden. Das 2,3-Dimethylbutan-2,3-diol-hydrat kann wie oben beschrieben abgetrennt werden. Als Mutterlauge hinterbleibt eine flüssige, im wesentlichen unumgesetzte 2,3-Dimethylbutene enthaltende Phase, wenn das vorhandene Wasser vollständig zur Bildung von 2,3-Dimethylbutan-2,3-diol-hydrat verbraucht wird. Ist mehr Wasser vorhanden, so hinterbleibt eine Mutterlauge, die neben der im wesentlichen unumgesetzte 2,3-Dimethylbutene enthaltenden organischen Phase noch eine wäßrige Phase enthält. Die organische und gegebenenfalls die wäßrige Phase können, gegebenenfalls nach Auftrennung in eine organische und eine wäßrige Phase, wie oben beschrieben, weiter aufgearbeitet und in die Reaktion zurückgeführt werden.

Das so erhaltene 2,3-Dimethylbutan-2,3-diol-hydrat kann bereits als solches für viele Zwecke verwendet werden, beispielsweise zur Umlagerung in Pinakolon (siehe z. B. Römpps Chemie Lexikon, Stuttgart 1974, Seite 2702). Falls erwünscht kann man aus dem 2,3-Dimethylbutan-2,3-diol-hydrat durch Abtrennung von Wasser 2,3-Dimethylbutan-2,3-diol erhalten. Die Abtrennung des Hydrat-Wassers kann beispielsweise durch azeotrope Entwässerung erfolgen.

Das erfindungsgemäße Verfahren ermöglicht eine einfache und wirtschaftliche Herstellung von 2,3-Dimethylbutan-2,3-diol. Die erzielbaren Selektivitäten der Umsetzung sind hoch, die Verwendung von im Vergleich zu Sauerstoff oder Luft teurem Wasserstoffperoxid wird vermieden, Katalysatoren werden nicht benötigt und die Reaktionszeiten sind kurz.

Es ist ausgesprochen überraschend, daß mit dem erfindungsgemäßen Verfahren diese Vorteile realisierbar sind, denn im Hinblick auf die eingangs beschriebenen Literaturstellen J. Org. Chem. 37, 2881 bis 2884 (1972) und aus JP-OS 65 087/78 konnte nicht erwartet werden, daß 2,3-Dimethylbutan-2,3-diol ohne Katalysatoren und bei kurzen Reaktionszeiten hergestellt werden kann.

Das erfindungsgemäße Verfahren wird durch die folgenden Beispiele erläutert, ohne es darauf einzuschränken. Sämtliche Prozentangaben stellen, soweit nichts anderes gesagt wird, Gewichtsprozente dar.

### Beispiel 1

In einem Edelstahl-Autoklaven, der mit einem mechanischen Rührer, einem Thermometer und einem Ventil versehen war, wurden 255 g eines Gemisches, bestehend aus 98% 2,3-Dimethyl-2-buten, 1% 2,3-Dimethyl-1-buten und 1% n-Hexan, sowie 108 g Wasser gegeben. Der Autoklav wurde unter Rühren (400 U/Min.) auf 100° C erwärmt. Unter weiterem Rühren wurden 25 bar Luft ausgedrückt und weitere 15 Minuten bei 100° C gerührt. Dann wurde der Autoklav auf Raumtemperatur unter Rühren abgekühlt und die Reaktionsprodukte analysiert. Die Auswertung ergab 3% 2,3-Dimethylbuten-Umsatz mit 89% Selektivität, bezogen auf hergestelltes 2,3-Dimethylbutan-2,3-diol.

### Beispiel 2

Es wurde verfahren wie in Beispiel 1, jedoch wurde der Autoklav nur auf 80° C erwärmt. Unter Rühren (400 U/Min.) wurden in Abständen von 10 Minuten fünfzehnmal 5 bar Sauerstoff aufgedrückt. Nach weiteren 15 Minuten wurde der Autoklav auf Raumtemperatur abgekühlt und die Reaktionsprodukte analysiert. Die Auswertung ergab 35% 2,3-Dimethylbuten-Umsatz mit 78% Selektivität, bezogen auf hergestelltes 2,3-Dimethylbutan-2,3-diol.

### Beispiel 3

In den im Beispiel 1 beschriebenen Autoklaven wurden 312 g eines Gemisches, bestehend aus 90% 2,3-Dimethyl-2-buten, 8,5% 2,3-Dimethyl-1-buten und 1,5% 2,3-Dimethylbutan, sowie 58 g einer wäßrigen Kaliumcarbonat-Lösung mit einem pH-Wert von 9 gegeben. Nach Erwärmen auf 50° C wurde unter Rühren (600 U/Min.) 80 bar eines Gasgemisches, bestehend aus 10% Sauerstoff und 90% Kohlendioxid, aufgedrückt und 30 Minuten nachgerührt. Nach Abkühlen auf Raumtemperatur wurden die Reaktionsprodukte analysiert. Die Auswertung ergab 1,8% 2,3-Dimethylbuten-Umsatz mit 72% Selektivität, bezogen auf hergestelltes 2,3-Dimethylbutan-2,3-diol.

### Beispiel 4

In den im Beispiel 1 beschriebenen Autoklaven wurden 51 g eines Gemisches bestehend aus 80% 2,3-Dimethyl-2-buten, 15% 2,3-Dimethyl-1-buten, 2% 2,3-Dimethylbutan und 3% n-Hexan sowie 412 g Wasser gegeben. Nach Aufheizen auf 125° C wurden unter Rühren (1000 U/Min.) 20 bar eines aus 50% Sauerstoff und 50% Stickstoff bestehenden Gasgemisches und nach weiteren 5 Minuten 3 bar reiner Sauerstoff aufgedrückt. Es wurde bei 125° C 10 Minuten nachgerührt. Der Autoklav wurde auf Raumtemperatur abgekühlt und die Reaktionsprodukte analysiert. Die Auswertung ergab 41% 2,3-Dimethylbuten-Umsatz mit 65% Selektivität, bezogen auf hergestelltes 2,3-Dimethylbutan-2,3-diol.

### Beispiel 5

In den im Beispiel 1 beschriebenen Autoklaven wurden 208 g eines Gemisches bestehend aus

95% 2,3-Dimethyl-2-buten, 3% 2,3-Dimethyl-1-buten, 1,5% 2,3-Dimethylbutan und 0,5% n-Hexan, sowie 83 g Wasser gegeben. Nach Erwärmen auf 117°C wurde unter Rühren (680 U/Min.) in Abständen von 10 Minuten jeweils 2,5 bar Sauerstoff 10mal aufgedrückt und anschließend 5 Minuten nachgerührt. Nach Abkühlen des Autoklaven auf 50°C und Druckentspannung über eine auf −70°C gekühlte Kühlfalle wurden zwei flüssige Phasen erhalten, die getrennt wurden. Die organische Phase wurde nach Destillation erneut zur Oxidation mit Sauerstoff eingesetzt. Die wäßrige Phase wurde auf +8°C abgekühlt und das sich nach Animpfen kristallin abscheidende 2,3-Dimethylbutan-2,3-diol-hydrat durch Zentrifugieren abgetrennt. Die Versuchsauswertung ergab 16,3% 2,3-Dimethylbuten-Umsatz mit 77% Selektivität, bezogen auf hergestelltes 2,3-Dimethylbutan-2,3-diol.

Beispiel 6

Es wurde verfahren wie in Beispiel 5, jedoch wurde nach dem letzten Aufdrücken von 2,5 bar Sauerstoff der Autoklav auf 15°C abgekühlt. Die Reaktionsmischung wurde nach Druckentspannung über eine mit Trockeneis gekühlte Kühlfalle in feste und flüssige Bestandteile durch Filtration getrennt, wobei der so erhaltene Feststoff kristallines 2,3-Dimethylbutan-2,3-diol-hydrat war. Die flüssigen Bestandteile wurden in einem Phasenabscheider in zwei flüssige Phasen getrennt. Die organische Phase wurde nach Rektifikation in einer Glockenbodenkolonne und die wäßrige Phase nach abdestillieren von niedriger als Wasser siedenden organischen Bestandteilen in die Reaktionszone zur erneuten Umsetzung zurückgeführt. Die Versuchsauswertung ergab 14,7% 2,3-Dimethylbuten-Umsatz mit 75% Selektivität, bezogen auf hergestelltes 2,3-Dimethylbutan-2,3-diol.

Beispiel 7

In eine aus 4 gerührten Druckreaktoren von je 300 ml Inhalt bestehende Kaskade wurden 700 g/h eines Gemisches bestehend aus 97% 2,3-Dimethyl-2-buten, 1,3% 2,3-Dimethyl-1-buten, 0,8% 2,3-Dimethylbutan und 0,9% n-Hexan, sowie 230 g/h Wasser eingegeben. Der erste und zweite Reaktor der Kaskade wurde bei 120°C betrieben, der dritte bei 115°C und der vierte bei 110°C. Nach Erreichen der vorgegebenen Reaktionstemperaturen wurde auf jeden Reaktor über ein Ventil soviel Sauerstoff aufgedrückt, daß der Gesamtdruck in der Kaskade 12 bar betrug. Das Reaktionsgemisch wurde nach der Kaskade druckentspannt unter Zusatz von Stickstoff, um explosionsfähige Gasgemische auszuschließen. Danach wurde das Reaktionsgemisch auf 52°C abgekühlt und in einem Abscheider in zwei flüssige Phasen aufgetrennt. Die organische Phase wurde nach Rektifikation in einer Siebbodenkolonne zur Aufkonzentrierung von nicht umgesetzten 2,3-Dimethylbutenen und nach Zusatz von frischem 2,3-Dimethylbuten-Gemisch in den ersten Reaktor der Kaskade zurückgeführt. Die wäßrige Phase wurde im Vakuum auf 12°C unter gleichzeitigem Abdestillieren von tiefer als Wasser siedenden organischen Bestandteilen abgekühlt. Das sich nach Animpfen kristallin abscheidende 2,3-Dimethylbutan-2,2-diol-hydrat wurde durch Abzentrifugieren abgetrennt. Die abdestillierten organischen Bestandteile wurden einer biologischen Klärung zugeführt. Die verbleibende wäßrige Mutterlauge wurde nach Zusatz von Frischwasser in den Prozeß zurückgeführt.

Die analytische Auswertung ergab 12% 2,3-Dimethylbuten-Umsatz mit einer Selektivität von 73%, bezogen auf hergestelltes 2,3-Dimethylbutan-2,3-diol-hexahydrat. Man erhielt pro Stunde 162 g kristallines 2,3-Dimethylbutan-2,3-diol-hexahydrat.

Beispiel 8

In einem Edelstahl-Autoklaven, der mit einem Begasungsrührer, einem Thermometer und einem Ventil versehen war, wurden 255 g eines Gemisches, bestehend aus 98% 2,3-Dimethyl-2-buten, 1% 2,3-Dimethyl-1-buten und 1% n-Hexan, sowie 108 g Wasser gegeben. Der Autoklav wurde unter Rühren (1000 U/Min.) auf 120°C erwärmt. Unter weiterem Rühren wurden 26 bar Luft aufgedrückt und weitere 15 Minuten bei 120°C gerührt. Während dieser Zeit wurde die verbrauchte Sauerstoffmenge kontinuierlich nachgeliefert. Dann wurde der Autoklav auf Raumtemperatur unter Rühren abgekühlt und die Reaktionsprodukte analysiert. Die Auswertung ergab 83% 2,3-Dimethylbuten-Umsatz mit 78% Selektivität, bezogen auf hergestelltes 2,3-Dimethylbutan-2,3-diol.

Beispiel 9

In einem Edelstahl-Autoklaven, der mit einem Begasungsrührer, einem Thermometer und einem Ventil versehen war, wurden 255 g eines Gemisches, bestehend aus 98% 2,3-Dimethyl-2-buten, 1% 2,3-Dimethyl-1-buten und 1% n-Hexan, sowie 108 g Wasser gegeben. Der Autoklav wurde unter Rühren (2000 U/Min.) auf 110°C erwärmt. Unter weiterem Rühren wurden 28 bar Luft aufgedrückt und weitere 30 Minuten bei 110°C gerührt. Während dieser Zeit wurde die verbrauchte Sauerstoffmenge kontinuierlich nachgeliefert. Dann wurde der Autoklav auf Raumtemperatur unter Rühren abgekühlt und die Reaktionsprodukte analysiert. Die Auswertung ergab 93% 2,3-Dimethylbuten-Umsatz mit 76% Selektivität, bezogen auf hergestelltes 2,3-Dimethylbutan-2,3-diol.

**Patentansprüche**

1. Verfahren zur Herstellung von 2,3-Dimethylbutan-2,3-diol, dadurch gekennzeichnet, daß man 2,3-Dimethylbutene mit Sauerstoff oder einem Sauerstoff enthaltenden Gas in Gegenwart von 0,01 bis 20 Gew.-Teilen Wasser (bezogen auf eingesetzte 2,3-Dimethylbutene) bei Temperaturen von 40 bis 180° C und Verweilzeiten von 5 bis 150 Minuten ohne Zusatz von Katalysatoren umsetzt.

2. Verfahren nach Anspruch 1, dadurch gekennzeichnet, daß man die Umsetzung bei Drukken im Bereich 0,9 bis 90 bar durchführt.

3. Verfahren nach Anspruch 1 und 2, dadurch gekennzeichnet, daß man 2,3-Dimethyl-2-buten, 2,3-Dimethyl-1-buten, beliebige Gemische aus 2,3-Dimethyl-2-buten und 2,3-Dimethyl-1-buten oder beliebige Gemische aus 2,3-Dimethyl-2-buten und/oder 2,3-Dimethyl-1-buten mit Inerten einsetzt.

4. Verfahren nach Anspruch 1 bis 3, dadurch gekennzeichnet, daß man Luft, Sauerstoff enthaltende Gasgemische, die über 50% Sauerstoff enthalten oder reinen Sauerstoff einsetzt.

5. Verfahren nach Anspruch 1 bis 4, dadurch gekennzeichnet, daß man den Sauerstoff in mehreren Teilmengen nacheinander zugibt.

6. Verfahren nach Anspruch 1 bis 5, dadurch gekennzeichnet, daß man mit basisch reagierenden Verbindungen versetztes Wasser einsetzt.

7. Verfahren nach Anspruch 1 bis 6, dadurch gekennzeichnet, daß man bei Temperaturen von 60 bis 160° C arbeitet.

8. Verfahren nach Anspruch 1 bis 7, dadurch gekennzeichnet, daß man das erhaltene Reaktionsgemisch auf Temperaturen unterhalb des Siedepunkts der 2,3-Dimethylbutene abkühlt und den Druck entspannt.

9. Verfahren nach Anspruch 8, dadurch gekennzeichnet, daß man das Reaktionsgemisch nach Druckentspannung zunächst auf Temperaturen oberhalb des Schmelzpunktes von 2,3-Dimethylbutan-2,3-diol-hydrat abkühlt, so ein flüssiges 2-Phasen-Gemisch erhält, die organische Phase in die Reaktion zurückführt, die wäßrige Phase auf Temperaturen unterhalb 48° C bis —10° C abkühlt, das sich kristallin abscheidende 2,3-Dimethylbutan-2,3-diol-hydrat abtrennt und die verbleibende Mutterlauge ganz oder teilweise in die Reaktion zurückführt.

10. Verfahren nach Anspruch 8, dadurch gekennzeichnet, daß man das Reaktionsgemisch nach Druckentspannung auf Temperaturen unterhalb des Schmelzpunktes von 2,3-Dimethylbutan-2,3-diol-hydrat abkühlt, das kristalline 2,3-Dimethylbutan-2,3-diol-hydrat abtrennt und die Mutterlauge, gegebenenfalls nach Auftrennung in eine organische und eine wäßrige Phase, in die Reaktion zurückführt.

**Claims**

1. Process for the preparation of 2,3-dimethylbutane-2,3-diol, characterised in that 2,3-dimethylbutane are reacted with oxygen or an oxygen-containing gas in the presence of 0.01 to 20 parts by weight of water (relative to 2,3-dimethylbutenes employed), at temperatures of from 40 to 180° C and residence times of from 5 to 150 minutes, without the addition of catalysts.

2. Process according to claim 1, characterised in that the reaction is carried out at pressures in the range from 0.9 to 90 bars.

3. Process according to claim 1 and 2, characterised in that 2,3-dimethyl-2-butene, 2,3-dimethyl-1-butene, any desired mixtures of 2,3-dimethyl-2-butene and 2,3-dimethyl-1-butene, or any desired mixtures of 2,3-dimethyl-2-butene and/or 2,3-dimethyl-1-butene are employed with inert solvents.

4. Process according to claim 1 to 3, characterised in that air, oxygen-containing gas mixtures which contain over 50% of oxygen, or pure oxygen are employed.

5. Process according to claim 1 to 4, characterised in that the oxygen is added successively in several portions.

6. Process according to claim 1 to 5, characterised in that water in employed to which basic compounds have been added.

7. Process according to claim 1 to 6, characterised in that the reaction is carried out at temperatures of from 60 5o 160° C.

8. Process according to claim 1 to 7, characterised in that the reaction mixture obtained is colled to temperatures below the boiling point of the 2,3-dimethylbutenes and the pressure is relaesed.

9. Process according to claim 8, characterised in that after the pressure has been released the reaction mixture ist firstly cooled to temperatures above the melting point of 2,3-dimethylbutane-2,3-diol hydrate, a liquid 2-phase mixture is thus obtained, the organic phase is recycled into the reaction, the aqueous phase is cooled to temperatures below 48°C to —10°C, the 2,3-dimethylbutane-2,3-diol hydrate which separates out in crystalline form is separated off, and the residual mother liquor is completely or partially recycled into the reaction.

10. Process according to claim 8, characterised in that after the pressure has been released the reaction mixture is cooled to temperatures below the melting point of 2,3-dimethylbutane2,3-diol hydrate, the crystalline 2,3-dimethylbutane-2,3-diol hydrate is separated off and the mother liquor, if appropriate after separation into an organic and an aqueous phase, is recycled into the reaction.

**Revendications**

1. Procédé de frabrication du 2,3-diméthylbutane-2,3-diol, caractérisé en ce qu'on fait réagir des 2,3-diméthylbutènes avec de l'oxygène ou un gaz renferment de l'oxygène en présence de 0,01 à 20 parties en poids d'eau (par rapport aux

2,3-diméthylbutènes utilisés) à des températures de 40 à 180° C et avec des temps de séjour de 5 à 150 minutes, sans addition de catalyseurs.

2. Procédé selon la revendication 1, caractérisé en ce qu'on exécute la réaction sous des pressions dans l'intervalle de 0,9 à 90 bars.

3. Procédé selon les revendications 1 et 2, caractérisé en ce qu'on utilise du 2,3-diméthyl-2-butène, due 2,3-diméthyl-1-butène, des mélanges quelconques de 2,3-diméthyl-2-butène et de 2,3-diméthyl-1-butène ou des mélanges quelconques de 2,3-diméthyl-2-butène et/ou de 2,3-diméthyl-1-butène avec des inertes.

4. Procédé selon les revendications 1 à 3, caractérisé en ce qu'on utilise de l'air, des mélanges gazeux renfermant de l'oxygéne qui contiennent plus de 50% d'oxygéne, ou de l'oxygéne pur.

5. Procédé selon les revendications 1 à 4, caractérisé en ce qu'on ajoute l'oxygéne en plusieurs quantités partielles.

6. Procédé selon les revendications 1 à 5, caractérisé en ce qu'on utilise de l'eau additionnée de composés à réaction basique.

7. Procédé selon les revendications 1 à 6, caractérisé en ce qu'on opère à des températures entre 60 et 160° C.

8. Procédé selon les revendications 1 à 7, caractérisé en ce qu'on refroidit le mélange de réaction obtenu à des températures inférieures au point d'ébullition des 2,3-diméthylbutènes et l'on détend la pression.

9. Procédé selon la revendication 8, caractérisé en ce qu'on refroidit le mélange de réaction, après détente de la pression, d'abord à des températures supérieures au point de fusion de l'hydrate de 2,3-diméthylbutane-2,3-diol, en obtenant ainsi un mélange liquide à 2 phases, on renvoie la phase organique à la réaction, on refroidit le phase aquese à des températures inférieures à 49° C et allant jusqu'à —10° C, on sépare l'hydrate de 2,3-diméthylbutane-2,3-diol qui se dépose en cristaux et l'on renvoie la liqueur-mère restante en totalité ou en partie à la réaction.

10. Procédé selon la revendication 8, caractérisé en ce qu'on refroidit le mélange de réaction, après détente de la pression, à des températures inférieures au point de fusion de l'hydrate de 2,3-diméthylbutane-2,3-diol, on sépare l'hydrate de 2,3-diméthylbutane-2,3-diol cristallisé et l'on renvoir à la réaction la liqueur-mère, éventuellement après séparation en une phase organique et en une phase aqueuse.